# EUROPÄISCHE PATENTSCHRIFT

(11) **EP 0 820 992 B1**
(45) Veröffentlichungstag und Bekanntmachung des Hinweises auf die Patenterteilung: **13.03.2002**
(21) Anmeldenummer: 97111345.1
(22) Anmeldetag: 04.07.1997
(51) Int. Cl.: C07D 239/60

(54) **Verfahren zur Herstellung von 4,6-Bis(difluormethoxy)-Pyrimidinderivaten**
Process for the preparation of 4,6-bis(difluoromethoxy)-pyrimidine derivatives
Procédé pour la préparation de dérivés de 4,6-bis(difluorométhoxy)-pyrimidine

(30) Priorität: 24.07.1996 CH 184696
(43) Veröffentlichungstag der Anmeldung: 28.01.1998
(73) Patentinhaber: Lonza AG, 3930 Visp (CH)
(72) Erfinder: Naepfli, Andreas, 3902 Glis (Kanton Wallis) (CH); Roduit, Jean-Paul, Dr., 3979 Grône (Kanton Wallis) (CH); Wellig, Alain, 3986 Ried-Mörel (Kanton Wallis) (CH)
(74) Vertreter: Winter, Brandl, Fürniss, Hübner, Röss, Kaiser, Polte Partnerschaft

(56) Entgegenhaltungen:
- US-A- 4 692 524
- US-A- 4 900 827

## Beschreibung

Die Erfindung betrifft ein neues Verfahren zur Herstellung von 4,6-Bis-(difluormethoxy)pyrimidinderivaten ausgehend von 4,6-Dihydroxypyrimidindialkalimetallsalzen.

4,6-Bis(difluormethoxy)pyrimidinderivate sind wichtige Zwischenprodukte zur Herstellung von Herbiziden wie z. B. zur Herstellung von Sulfonylharnstoffen (US-PS 4 900 827).

Bisher sind mehrere Verfahren zur Herstellung von 4,6-Bis(difluormethoxy)-pyrimidinderivaten bekannt.
Die US-PS 4 900 827 beschreibt ein Verfahren zur Herstellung von 4,6-Bis-(difluormethoxy)pyrimidinderivaten, bei dem ein 4,6-Dihydroxypyrimidindialkalimetallsalz mit Chlordifluormethan, gelöst in Acetonitril oder Aceton, in Gegenwart von 0,05 bis 1,1 mol Wasser pro mol 4,6-Dihydroxypyrimidindialkalimetallsalz umgesetzt wird. Nachteilig bei diesem Verfahren ist, dass die Reaktion in einem heterogenen dreiphasigen System durchgeführt wird. D. h. Acetonitril oder Aceton stellt die flüssige Phase dar, das 4,6-Dihydroxypyrimidindialkalimetallsalz ist ein Feststoff und das Chlordifluormethan ist zum Teil gelöst und zum Teil in der Gasphase.

Die EP-A 0 468 069 beschreibt ein Verfahren zur Herstellung von 4,6-Bis(difluormethoxy)pyrimidinderivaten ausgehend von dem entsprechenden 4,6-Dihydroxypyrimidin durch Reaktion bei Normaldruck mit Chlordifluormethan in Gegenwart von Natriumhydroxid, gelöst in Dioxan. Nachteilig bei diesem Verfahren ist, dass eine beträchtliche Menge an Chlordifluormethan eingesetzt wird und dadurch das Verfahren nicht ökologisch ist. Zudem ist Dioxan als Lösungsmittel in der Produktion nicht geeignet.

Aufgabe der vorliegenden Erfindung war ein ökologischeres Verfahren zur Herstellung von 4,6-Bis(difluormethoxy)pyrimidinderivaten zur Verfügung zu stellen, welches unter milderen Reaktionsbedingungen wie tieferer Temperatur durchgeführt werden kann.

Diese Aufgabe wird mit dem neuen Verfahren gemäss Patentanspruch 1 gelöst.

Erfindungsgemäss wird das Verfahren derart durchgeführt, dass man ein 4,6-Dihydroxypyrimidindialkalimetallsalz der allgemeinen Formel 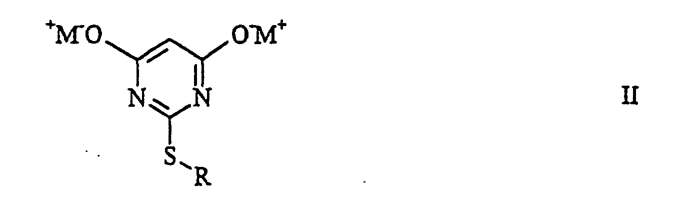 unter Druck mit Chlordifluormethan, in einem Lösungsmittel aus der Gruppe der Ketone, in Gegenwart einer Base, eines Phasen-Transfer-Katalysators und in Gegenwart von 40 bis 100 mol Wasser pro mol des 4,6-Dihydroxy-pyrimidindialkalimetallsalzes der allgemeinen Formel II, zum Endprodukt der Formel 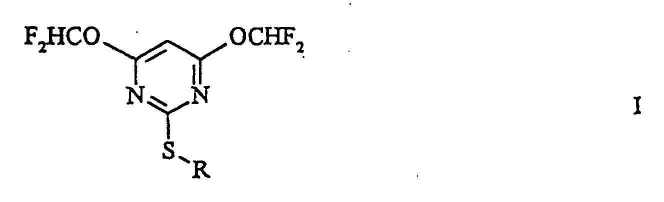 umsetzt.

Der Substituent R bedeutet entweder C₁₋₄-Alkyl oder gegebenenfalls mit C₁₋₄-Alkyl, Halogen, Nitro oder C₁₋₄-Alkoxy substituiertes Phenyl oder Benzyl. C₁₋₄-Alkyl kann Methyl, Ethyl, Propyl, Isopropyl, Butyl, Isobutyl oder tert-Butyl bedeuten. Als Halogen kommen beispielsweise F, Cl, Br oder I und als C₁₋₄-Alkoxy Methoxy, Ethoxy, Propoxy oder Butoxy in Betracht. Vorzugsweise bedeutet R Methyl.

Die Edukte, die 4,6-Dihydroxypyrimidindialkalimetallsalze sind kommerziell erhältlich oder können gemäss der EP-A 0 529 631 synthetisiert werden.

Geeignete Lösungsmittel aus der Gruppe der Ketone sind bspw. Aceton, Methylethylketon, Diethylketon, Methylisopropylketon oder Methylisobutylketon.Vorzugsweise wird als Lösungsmittel Aceton verwendet.

Als Base kann ein Alkalimetall-oder Erdalkalimetallhydroxid verwendet werden. Als Alkalimetallhydroxid kann Lithium-,Natrium-oder Kaliumhydroxid eingesetzt werden. Als Erdalkalimetallhydroxid kann Calcium- oder Magnesiumhydroxid eingesetzt werden. Vorzugsweise wird als Base Natriumhydroxid eingesetzt.

Zweckmässig wird die Base in einer Menge von 2 bis 8 mol, vorzugsweise von 4,5 bis 6 mol, pro mol 4,6-Dihydroxypyrimidindialkalimetallsalz eingesetzt.

Als Phasen-Transfer-Katalysatoren sind generell quartäre Ammoniumsalze geeignet. Bevorzugte Phasen-Transfer-Katalysatoren sind Benzyltrimethylammoniumchlorid, Tetrabutylammoniumchlorid, Tetramethylammoniumbromid und Tetramethylammoniumchlorid. Insbesondere wird als Phasen-Transfer-Katalysator Tetramethylammoniumchlorid verwendet.

Die Phasen-Transfer-Katalysatoren können in einer Menge von 0,05 bis 0,2 mol, vorzugsweise von 0,1 bis 0,15 mol, pro mol 4,6-Dihydroxypyrimidindialkalimetallsalz eingesetzt werden.

Das Chlordifluormethan kann in einer Menge von 2 bis 6 mol, vorzugsweise von 3 bis 4 mol, pro mol 4,6-Dihydroxypyrimidindialkalimetallsalz eingesetzt werden.

Die Umsetzung erfolgt in Gegenwart von 40 bis 100 mol Wasser, vorzugsweise von 60 bis 90 mol, pro mol 4,6-Dihydroxypyrimidindialkalimetallsalz.

Die Umsetzung wird unter erhöhtem Druck, vorzugsweise bei einem Druck von 1 bis 6 bar, insbesondere von 2 bis 5 bar, durchgeführt.

Zweckmässig wird die Umsetzung bei einer Temperatur von 40 bis 60 °C, vorzugsweise von 45 bis 55 °C, durchgeführt.

Mit dem erfindungsgemässen Verfahren ist es möglich die 4,6-Bis(difluormethoxy)-pyrimidinderivate mit einer geringen Menge an Chlordifluormethan in guter Ausbeute herzustellen. Ein weiterer Vorteil sind die milden Reaktionsbedingungen wie tiefe Temperatur und die geringe Reaktionszeit.

### Beispiel

### Herstellung von 4,6-Bis(difluormethoxy)-2-methylthiopyrimidin

In einem Autoklaven wurden bei 20 °C feuchtes Methylthiobarbitursäurenatriumsalz (242 g, 58,3 % Gehalt; 0,698 mol), Wasser (381 g; 21 mol), Tetramethylammoniumchlorid (9,7 g; 0,0878 mol) und Aceton (261 g) vorgelegt. Der Autoklav wurde geschlossen und mit Stickstoff begast. Das Gemisch wurde unter starkem Rühren auf 46 °C erhitzt und anschliessend wurde Chlordifluormethan (115,3 g; 1,333 mol) bei einem Druck von 3,1 bar eingeleitet.
Innerhalb 1,5 h wurde 25%ige NaOH (246.6 g, 1,541 mol) bei konstanter Temperatur zudosiert. Anschliessend wurde nochmals Aceton (261 g), Chlordifluormethan (76,8 g; 0.889 mol) und 25%ige NaOH (349 g; 2,181 mol) innerhalb 2 h bei 46 °C hinzugegeben. Am Ende der Reaktion betrug der Druck 4,3 bar. Das Reaktionsgemisch wurde noch 1 h nachgerührt und dann auf 20 °C abgekühlt. Nach Entspannung (Absorbieren des Chlordifluormetans im Aceton) wurde die organische Phase getrennt, Aceton abdestilliert und der Rückstand mit Chlorbenzol (275 g) und Wasser (100 g) extrahiert. Insgesamt wurden 106,3 g 4,6-Bis(difluormethoxy)-2-methylthiopyrimidin, entsprechend einer Ausbeute von 59 %,erhalten.

## Patentansprüche

1. Verfahren zur Herstellung von 4,6-Bis(difluormethoxy)pyrimidinderivaten der allgemeinen Formel 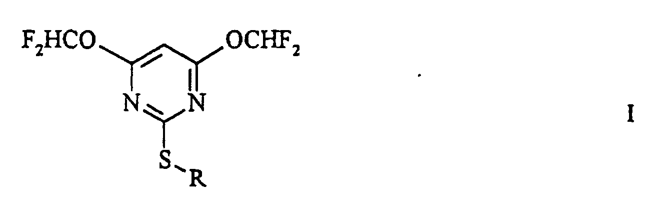 worin R C₁₋₄-Alkyl oder gegebenenfalls mit C₁₋₄-Alkyl, Halogen, Nitro oder C₁₋₄-Alkoxy substituiertes Phenyl oder Benzyl bedeutet, **dadurch gekennzeichnet, dass** man ein 4,6-Dihydroxypyrimidindialkalimetallsalz der allgemeinen Formel 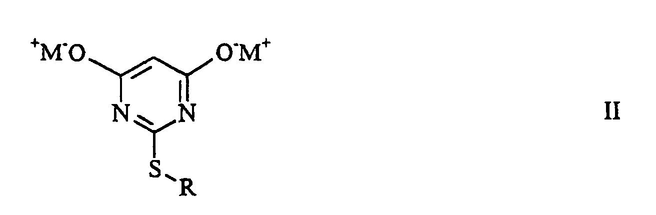 worin R die genannte Bedeutung hat und M ein Alkalimetallatom bedeutet, unter Druck mit Chlordifluormethan, in einem Lösungsmittel aus der Gruppe der Ketone, in Gegenwart einer Base, eines Phasen-Transfer-Katalysators und in Gegenwart von 40 bis 100 mol Wasser pro mol des 4,6-Dihydroxypyrimidindialkalimetallsalzes der allgemeinen Formel II, zum Endprodukt der Formel I umsetzt.

2. Verfahren nach Patentanspruch 1, **dadurch gekennzeichnet, dass** man als Lösungsmittel aus der Gruppe der Ketone Aceton einsetzt.

3. Verfahren nach einem der Patentansprüche 1 oder 2, **dadurch gekennzeichnet, dass** man als Base ein Alkalimetallhydroxid verwendet.

4. Verfahren nach mindestens einem der Patentansprüche 1 bis 3, **dadurch gekennzeichnet, dass** man 2 bis 6 mol Chordifluormethan pro mol 4,6-Dihydroxypyrimidindialkalimetallsalz einsetzt.

5. Verfahren nach mindestens einem der Patentansprüche 1 bis 4, **dadurch gekennzeichnet, dass** man die Umsetzung bei einer Temperatur von 40 bis 60 °C durchführt.

6. Verfahren nach mindestens einem der Patentansprüche 1 bis 5, **dadurch gekennzeichnet, dass** man die Umsetzung bei einem Druck von 1 bis 6 bar durchführt.

7. Verfahren nach mindestens einem der Patentansprüche 1 bis 6, **dadurch gekennzeichnet, dass** man als Phasen-Transfer-Katalysator Tetramethylammoniumchlorid einsetzt.

## Claims

1. Process for the production of 4,6-bis(difluoromethoxy)pyrimidine derivatives of the general formula 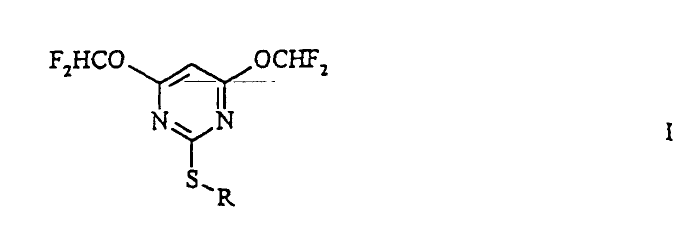 wherein R stands for C₁₋₄ alkyl, or optionally phenyl or benzyl substituted with C₁₋₄ alkyl, halogen, nitro or C₁₋₄ alkoxy, **characterised in that** a 4,6-dihydroxypyrimidine dialkali metal salt of the general formula 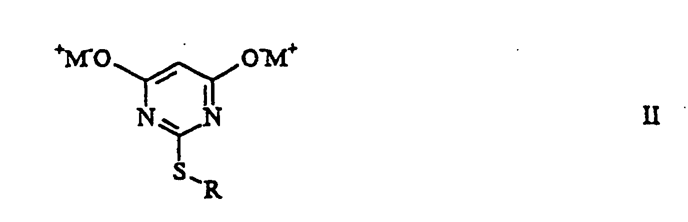 wherein R has the above meaning and M stands for an alkali metal atom, is reacted under pressure with chlorodifluoromethane, in a solvent from the group of the ketones, in the presence of a base, a phase transfer catalyst and in the presence of 40 to 100 moles of water per mole of the 4,6-dihydroxypyrimidine dialkali metal salt of the general formula II, to give the end product of formula I.

2. Process according to claim 1, **characterised in that** acetone is used as the solvent from the group of the ketones.

3. Process according to one of claims 1 or 2, **characterised in that** an alkali metal hydroxide is used as the base.

4. Process according to at least one of claims 1 to 3, **characterised in that** 2 to 6 moles of chlorodifluoromethane are used per mole of 4,6-dihydroxypyrimidine dialkali metal salt.

5. Process according to at least one of claims 1 to 4, **characterised in that** the reaction is performed at a temperature of 40 to 60°C.

6. Process according to at least one of claims 1 to 5, **characterised in that** the reaction is performed under a pressure of 1 to 6 bar.

7. Process according to at least one of claims 1 to 6, **characterised in that** tetramethylammonium chloride is used as the phase transfer catalyst.

## Revendications

1. Procédé de préparation de dérivés de la 4,6-bis(difluorométhoxy)-pyrimidine de formule générale 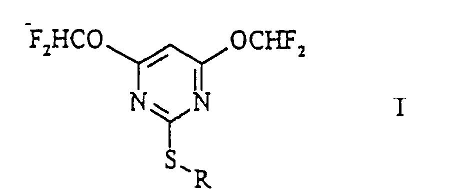 dans laquelle R représente un reste alkyle en C₁-C₄ ou un reste phényle ou benzyle éventuellement substitué par alkyle en C₁-C₄, halogéno, nitro ou alcoxy en C₁-C₄, **caractérisé en ce que** l'on fait réagir un sel de métal alcalin de 4,6-dihydroxypyrimidine de formule générale 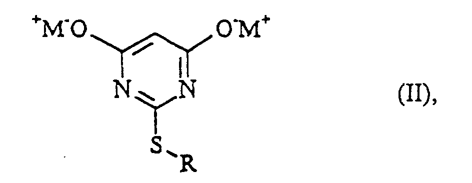 dans laquelle R a la signification indiquée et M représente un atome de métal alcalin, avec du chlorodifluorométhane sous pression, dans un solvant du groupe des cétones, en présence d'une base et d'un catalyseur de transfert de phase et en présence de 40 à 100 mol d'eau par mol du sel de métal alcalin de 4,6-dihydroxypyrimidine de formule générale II, pour obtenir le produit final de formule I.

2. Procédé selon la revendication 1, **caractérisé en ce que** l'on utilise de l'acétone comme solvant du groupe des cétones.

3. Procédé selon l'une des revendications 1 ou 2, **caractérisé en ce que** l'on utilise comme base un hydroxyde de métal alcalin.

4. Procédé selon au moins l'une des revendications 1 à 3, **caractérisé en ce que** l'on utilise 2 à 6 mol de chlorodifluorométhane par mol de sel de métal alcalin de 4,6-dihydroxypyrimidine.

5. Procédé selon au moins l'une des revendications 1 à 4, **caractérisé en ce que** l'on effectue la réaction à une température de 40 à 60°C.

6. Procédé selon au moins l'une des revendications 1 à 5, **caractérisé en ce que** l'on effectue la réaction sous une pression de 1 à 6 bars.

7. Procédé selon au moins l'une des revendications 1 à 6, **caractérisé en ce que** l'on utilise du chlorure de tétraméthylammonium comme catalyseur de transfert de phase.
